(19) Europäisches Patentamt
European Patent Office
Office européen des brevets

(11) Veröffentlichungsnummer : **0 055 427**
**B1**

(12) **EUROPÄISCHE PATENTSCHRIFT**

(45) Veröffentlichungstag der Patentschrift : **02.05.84**

(21) Anmeldenummer : **81110565.9**

(22) Anmeldetag : **18.12.81**

(51) Int. Cl.³ : **C 07 C 49/16**, C 07 C 49/227, C 07 C 45/42

# (54) Verfahren zur Herstellung von Monochlormethylketonen.

(30) Priorität : **30.12.80 DE 3049461**

(43) Veröffentlichungstag der Anmeldung : **07.07.82 Patentblatt 82/27**

(45) Bekanntmachung des Hinweises auf die Patenterteilung : **02.05.84 Patentblatt 84/18**

(84) Benannte Vertragsstaaten : **AT BE CH DE FR GB IT LI NL**

(56) Entgegenhaltungen :
**DE-A- 2 716 895**
**DE-B- 2 716 896**
**US-A- 2 988 537**

(73) Patentinhaber : **BAYER AG**
**Konzernverwaltung RP Patentabteilung**
**D-5090 Leverkusen 1 Bayerwerk (DE)**

(72) Erfinder : **Jautelat, Manfred, Dr.**
**Muellersbaum 28**
**D-5093 Burscheid 1 (DE)**
Erfinder : **Arlt, Dieter, Prof. Dr.**
**Rybniker Strasse 2**
**D-5000 Koeln 80 (DE)**
Erfinder : **Jäger, Gerhard, Dr.**
**Gellertstrasse 18**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 055 427 B1

Verfahren zur Herstellung von Monochlormethylketonen

Die vorliegende Erfindung betrifft ein neues Verfahren zur Herstellung von teilweise bekannten Monochlormethylketonen, die als Zwischenprodukte für die Synthese von fungizid wirksamen Azol-Derivaten verwendet werden können.

Es ist bereits bekannt, daß man Chlormethylketone durch Chlorierung von Methylketonen erhält (Houben-Weyl, « Methoden der Org. Chemie », Band 7/2 c, Seite 2162, Georg Thieme Verlag Stuttgart (1977)). Dieses Verfahren hat jedoch den Nachteil, daß in vielen Fällen die Produkte nicht einheitlich sind, weil eine Höherchlorierung zu Dichlor- und Trichlorketonen in 1- und 3-Stellung des Ketons erfolgt. Sind weitere, gegen Halogen sensitive funktionelle Gruppe wie z. B. eine Doppelbindung im Keton vorhanden, so ist diese Methode nicht mehr anwendbar.

Es wurde nun gefunden, daß man die teilweise bekannten Monochlormethylketone der allgemeinen Formel

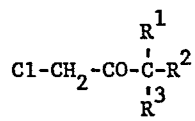

(I)

in welcher $R^1$, $R^2$ und $R^3$ wie nachfolgend definiert sind erhält, wenn man 1,1-Dichloralkene der Formel

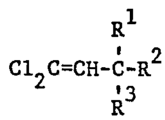

(II)

mit Phenolaten der Formel

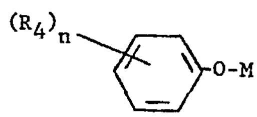

(III)

in welcher

$R^4$ wie nachfolgend definiert ist

n für die Zahlen 0, 1, 2 oder 3 steht,

M für ein Äquivalent eines Alkali- oder Erdalkalimetallions steht,

umsetzt, und hernach das dabei erhaltene Reaktionsprodukt einer sauren Hydrolyse unterwirft.

Die Verbindungen der allgemeinen Formel (I) sind Zwischenprodukte für die Herstellung von Pflanzenschutzmitteln.

Nach bisherigen Kenntnissen führt die Umsetzung von Vinylidenchlorid mit Alkoholaten zu Ketenacetalen und Orthoessigsäureestern (J. Org. Chem. *29,* 2773 (1964) u. J. Org. Chem. *30,* 3926 (1965)) ; außerdem ist bekannt, daß die Umsetzung von 1,1-Dichlor-3,3-dimethyl-1,4-pentadien und Alkoholaten zu 3,3-Dimethyl-4-pentensäure führt (Deutsche Patentanmeldung P 30 29 270.7 vom 01.08.1980 [Le A 20 475]). Es ist deshalb als ausgesprochen überraschend zu bezeichnen, daß nach dem erfindungsgemäßen Verfahren aus 1,1-Dichloralkenen der Formel (II) mit Phenolaten der Formel (III) und nachfolgender saurer Hydrolyse der Reaktionsprodukte die Monochlormethylketone der allgemeinen Formel (I) erhalten werden.

Das erfundene Verfahren weist eine Reihe von Vorteilen auf. So sind die Ausgangsstoffe, die 1,1-Dichloralkene der Formel (II), aus der Addition von Alkylhalogeniden an Vinylidenchlorid unter Lewis-Säure-Katalyse (sogenannte Schmerling-Reaktion, vgl. J. Am. Chem. Soc. 74, 2885 (1952)) gut und einfach zugänglich. Die Umsetzung der Verbindungen der Formel (II) mit den Phenolaten der Formel (III) verläuft in guten Ausbeuten und die nachfolgende saure Hydrolyse liefert daraus in selektiver Weise ausschließlich Monochlormethylketone der allgemeinen Formel (I). Auf diese Weise können auch ungesättigte Monochlormethylketone hergestellt werden. Die erfindungsgemäße Umsetzung stellt somit eine Bereicherung der Technik dar.

Verwendet man beispielsweise 1,1-Dichlor-3,3-dimethyl-1,4-pentadien und Natrium-phenolat als Ausgangsstoffe, so kann der Reaktionsablauf durch das folgende Formelschema wiedergegeben werden :

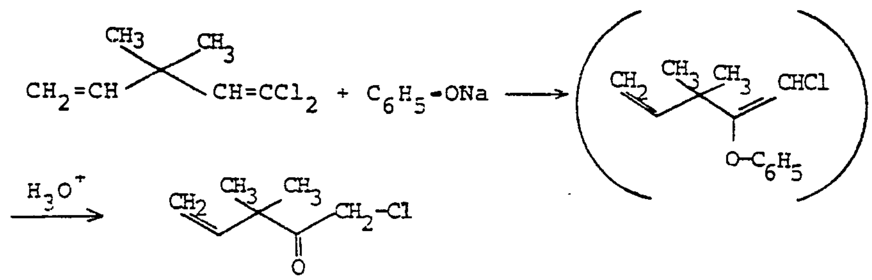

Die verwendeten Ausgangsstoffe sind durch die Formeln (II) und (III) allgemein definiert. In der Formel (II) stehen $R^1$ und $R^2$, die gleich oder verschieden sein können, für Alkyl mit bis zu 4 Kohlenstoffatomen, Alkenyl und Alkinyl mit jeweils 2 bis 4 Kohlenstoffatomen, und für Phenyl. Die genannten Gruppen können durch Halogen, Phenyl, Alkoxy mit bis zu 3 Kohlenstoffatomen oder Phenoxy substituiert sein. $R^1$ und $R^2$ können außerdem noch eine Alkylenkette mit 2 bis 7 Kohlenstoffatomen bilden, die gegebenenfalls durch Methyl-Gruppen und/oder Alkoxycarbonyl-Gruppen mit 1 bis 3 Kohlenstoffatomen substituiert sein kann. $R^3$ steht für Wasserstoff und Alkyl mit bis zu 10 Kohlenstoffatomen oder Aryl mit 6 bis 10 Kohlenstoffatomen. Die Alkyl- und Aryl-Reste können durch Halogen, Nitro, Alkoxy, Alkyl, Phenyl oder Cyano substituiert sein. Als Beispiele für die verwendeten 1,1-Dichloralkene der Formel (II) seien im einzelnen genannt :

1,1-Dichlor-3-methyl-1-buten, 1,1-Dichlor-3,3-dimethyl-1-buten, 1,1-Dichlor-3,3-dimethyl-1-penten, 1,1-Dichlor-3,3-dimethyl-1,4-pentadien, 1,1-Dichlor-3,3,4-trimethyl-1-penten, 1,1-Dichlor-3,3,5,5-tetra-1-hexen, 1,1-Dichlor-3,3-diethyl-1-penten, 1,1-Dichlor-3,3-dimethyl-1-octen, 1,1,4-Trichlor-3,3-dimethyl-1-buten, 1,1,5-Trichlor-3,3-dimethyl-1-penten, 1,1,4-Trichlor-3,3-dimethyl-1-penten, 1,1-Dichlor-3,3-dimethyl-5-fluor-1-penten, 1,1-Dichlor-3,3-dimethyl-5-methoxy-1-penten, 1,1-Dichlor-3,3-dimethyl-5-(p-chlorphenoxy)-1-penten, 1,1-Dichlor-3-methyl-3-(p-chlorphenyl)-1-buten, 1,1-Dichlor-1-buten, 1-(2,2-Dichlorvinyl)-1-methylcyclohexan, 1-(2,2-Dichlorvinyl)-1-ethyl-cyclopentan, 1-(2,2-Dichlorvinyl)-1-methylcyclopropan, 1,1,4-Trichlor-3,3-di-(chlormethyl)-1-buten, 1,1-Dichlor-3-methyl-3-(2,4-dichlorphenyl)-1-buten, 1,1-Dichlor-3,3-dimethyl-4-(2,4-dichlorphenyl)-1-buten, 1,1-Dichlor-3,3-dimethyl-pent-1-en-4-on, 1,1-Dichlor-3-(2,4-dichlorphenyl)-1-propen.

Die Herstellung der 1,1-Dichloralkene der Formel (II) ist bekannt, sie erfolgt durch Addition von Alkylhalogeniden an Vinylidenchlorid in Gegenwart von sauren Katalysatoren (J. Am. Chem. Soc. 74, 2885 (1952)) mit gleichzeitiger Abspaltung von Halogenwasserstoff.

Die weiterhin als Ausgangsprodukte benötigten Phenolate sind durch die Formel (III) allgemein definiert. In dieser Formel steht $R^4$ für Chlor und Fluor, für Alkyl und Alkoxy mit jeweils 1 bis 3 Kohlenstoffatomen und für Phenyl. Der Index n hat vorzugsweise die Werte 0, 1 und 2. Die Substituenten können sich in 2-, 3- und/oder 4-Stellung befinden. Als Metallionen kommen vorzugsweise die Ionen der Alkalimetalle, insbesondere Natrium- und Kaliumionen in Frage. Als Beispiele für die Phenolate seien die Natrium- und Kalium-Salze folgender Phenole genannt : 4-Chlorphenol, 2,4-Dichlorphenol, 2,4,6-Trichlorphenol, 4-Methylphenol, 4-Phenylphenol, 2,6-Dimethylphenol, 3-Methylphenol, 4-Methoxyphenol, 4-Fluorphenol, 4-Bromphenol, 2-Chlorphenol, 2-Fluorphenol, 2-Chlor-4-fluorphenol, 2,4-Difluorphenol, 2-Methyl-4-chlorphenol, 4-(p-Chlorphenyl)-phenol.

Die Phenolate der allgemeinen Formel (III) sind allgemein bekannte und laboratoriumsübliche Verbindungen.

Als Verdünnungsmittel kommen alle inerten organischen Lösungsmittel in Frage. Vorzugsweise werden polare Lösungsmittel, insbesondere Dimethylformamid, N-Methylpyrrolidon, Hexamethylphosphorsäuretriamid, Dimethylsulfoxid, 1,3-Dimethyl-2-imidazolidon, Tetramethylharnstoff, Sulfolan u. a., verwendet.

Die Reaktionstemperatur kann in einem größeren Bereich variiert werden. Im allgemeinen arbeitet man zwischen 50 und 250 °C, vorzugsweise zwischen 100 und 200 °C. Die Umsetzung kann sowohl bei Normaldruck im offenen System als auch unter Druck im Autoklaven ausgeführt werden.

Bei der Durchführung des erfindungsgemäßen Verfahrens setzt man 1 bis 4 Äquivalente Phenolat der Formel (III), vorzugsweise 2 bis 3 Äquivalente wasserfreies Phenolat, mit einem Äquivalent an 1,1-Dichloralken der Formel (II) um.

Die Umsetzung kann dabei in der Weise vorgenommen werden, daß man den aus 1,1-Dichloralken der Formel (II) und Phenolat der Formel (III) erhaltenen Phenylether der allgemeinen Formel

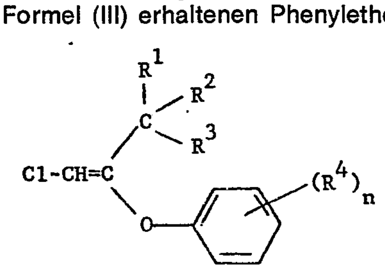

(IV)

in welcher $R^1$, $R^2$, $R^3$, $R^4$ und n die weiter oben angegebenen Bedeutungen besitzen, isoliert und — zum Beispiel durch fraktionierte Destillation — reinight, ehe man ihn durch saure Hydrolyse zu den Verbindungen der allgemeinen Formel (I) umsetzt.

Die nachfolgende Hydrolyse wird z. B. mit Mineralsäuren, vorzugsweise Schwefelsäure oder Salzsäure, und/oder mit organischen Säuren, wie Ameisensäure, Trifluoressigsäure, Oxalsäure, p-Toluolsulfonsäure oder Methansulfonsäure bei Temperaturen von + 20 bis 150 °C, vorzugsweise bei 40 bis 100 °C, ausgeführt. Als Lösungsvermittler kommen Alkohole wie Ethanol und Methanol, Ketone wie Aceton, oder Ether wie Dioxan in Betracht. Die Säuren werden im allgemeinen im Überschuß eingesetzt, sie können auch in wäßriger Verdünnung vorliegen.

Beide Reaktionsschritte können nicht nur getrennt, sondern auch nacheinander im sogenannten « Eintopf-Verfahren » ausgeführt werden, ohne daß der Phenylether der Formel (IV) isoliert wird.

Wie schon erwähnt, sind die nach dem erfindungsgemäßen Verfahren erhältlichen Monochlormethylketone der Formel (I) Zwischenprodukte zur Herstellung von fungizid wirksamen Azol-Derivaten. So kann man z. B. durch Umsetzung dieser Verbindungen mit Phenol oder Phenol-Derivaten (gemäß einer sogenannten Williamson-Synthese) die entsprechenden Phenoletherketone herstellen, letztere mit Halogenierungsmitteln zu den entsprechenden halogenierten Phenoletherketonen umsetzen, und endlich durch Umsetzung mit Azolen die entsprechenden Azolyl-Verbindungen erhalten (vgl. dazu die DE-PS 27 13 777 [Le A 17 981]). Diese Verbindungen besitzen bekanntlich eine gute fungizide Wirksamkeit (vgl. DE-PS 22 01 063 [Le A 14 118]).

Die folgenden Beispiele illustrieren das erfindungsgemäße Verfahren.

## Herstellungsbeispiele

Zwecks besserer Übersichtlichkeit werden die zunächst aus 1,1-Dichloralkenen der Formel (II) und Phenolaten der Formel (III) erhaltenen Phenolether der Formel (IV) mit *A* und nachfolgender Verbindungsnummer bezeichnet, z. B A1 und A2. Verschiedenartig hergestellte, jedoch ansonsten identische Verbindungen werden durch nachgestellte kleine Buchstaben (in Klammern) unterschieden, z. B. A1 (a) und A1 (b). Die durch Hydrolyse der Phenolether der Formel (IV) erhaltenen Monochlormethylketone der Formel (I) werden mit B und nachfolgender Verbindungsnummer bezeichnet, z. B. B1 und B2. Nachgestellte kleine Buchstaben in Klammern unterscheiden verschiedenartig hergestellte identische Verbindungen wie bei den A-Verbindungen oben angegeben.

### Beispiel A1 (a)

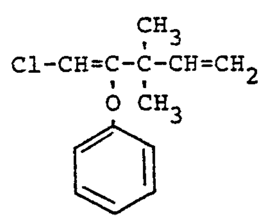

116 g (1 Mol) Natrium-phenolat und 82,5 g (0,5 Mol) 1,1-Dichlor-3,3-dimethyl-1,4-pentadien werden in 500 ml Dimethylformamid 8 Stunden unter Rückfluß erhitzt. Die Lösung wird mit Methylenchlorid verdünnt und mit verdünnter Natronlauge ausgeschüttelt. Nach Trocknen der Methylenchlorid-Phase über Natriumsulfat wird das Lösungsmittel im Vakuum abgezogen. Es bleiben 108,5 g (das sind 97 % der Theorie) Rohprodukt zurück, welches destilliert wird. Bei Kp 0,5 mm Hg/80-90 °C gehen 93,6 g (das sind 84 % der Theorie) 1-Chlor-3,3-dimethyl-2-phenoxy-1,4-pentadien über.

NMR ($CDCl_3$) : δ 1,25 (s, 6H), 4,9-6,2 ($—CH=CH_2$), 5,85 (s, 1H), 6,8-7,4 (m, 5H).

### Darstellung der Vorprodukte

3,3-Dimethyl-1,1,5-trichlor-1-penten

Unter Rühren und Kühlen auf – 10 °C werden in 2 300 g 1,1-Dichlor-ethen 20 g Aluminiumchlorid gelöst. Darauf werden innerhalb von 3 Stunden 1 286 g 1,3-Dichlor-3-methylbutan zugetropft und gleichzeitig in Abständen von 15 Minuten jeweils weitere 3 g Aluminiumchlorid zudosiert, wobei durch Kühlung eine Reaktionstemperatur zwischen 0° und + 5 °C eingehalten wird. Nach Beendigung der Reaktion werden 60 ml Essigsäure zum Reaktionsgemisch zugetropft. Danach wird das Produktgemisch über Natriumsulfat filtriert und anschließend in eine Destillationsapparatur eindosiert, wobei die Sumpftemperatur auf 120 °C gehalten und ein Druck von 1 mbar eingehalten wird ; das Destillat wird durch eine Trockeneis/Aceton-Mischung gekühlt und kondensiert. Anschließend wird das Rohdestillat an einer Vigreux-Kolonne im Vakuum fraktioniert. Man erhält 1 650 g 3,3-Dimethyl-1,1,5-trichlor-1-penten vom Kp 0,1 mm Hg/59-63 °C.

1,1-Dichlor-3,3-dimethyl-1,4-pentadien

Zu 1 l Chinolin und 83 g Kaliumcarbonat werden bei 225 bis 230 °C langsam 201,5 g (1 Mol) 3,3-Dimethyl-1,1,5-trichlor-1-penten eingetropft und über den Kopf einer Kolonne wird gleichzeitig Destillat abgenommen. Man steigert die Temperatur im Kolben bis zum Sieden des Chinolins und isoliert insgesamt 126 g Destillat, das nochmals fraktioniert wird. Beim Kp 20 mm Hg/59-53 °C erhält man 121 g (das sind 73 % der Theorie) 1,1-Dichlor-3,3-dimethyl-1,4-pentadien.

Beispiel B1 (a)

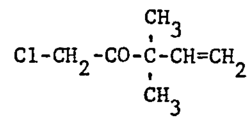

57 g (0,256 Mol) 1-Chlor-3,3-dimethyl-2-phenoxy-1,4-pentadien (siehe Beispiel A1 (a)) werden im Gemisch aus 250 ml Ameisensäure und 50 ml konz. Salzsäure während 1 Stunde auf 40 °C erwärmt. Dann wird mit 400 ml Methylenchlorid und Eis verdünnt und dreimal mit 2 n-Natronlauge ausgeschüttelt. Nach dem Trocknen der Methylenchloridphase über Natriumsulfat wird das Lösungsmittel entfernt. Es bleiben 36 g Produkt (das sind 96 % der Theorie) zurück, welches destilliert wird. Man erhält bei Kp 24 mm Hg/81-84 °C 32,2 g (das sind 86 % der Theorie) 1-Chlor-3,3-dimethyl-pent-4-en-2-on.

NMR ($CDCl_3$ : δ 1,3 (s, 6H), 4,35 (s, 2H), 5,0-6,2 ($—CH{=}CH_2$)

Beispiel A1 (b)

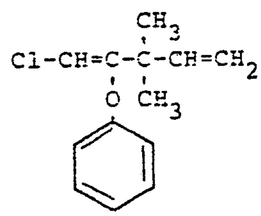

23,2 g (0,2 Mol) Natrium-phenolat und 16,5 g (0,1 Mol) 1,1-Dichlor-3,3-dimethyl-1,4-pentadien werden in 100 ml N-Methylpyrrolidon während 10 Stunden auf 180 °C erhitzt. Die Beispiel A1 (a) entsprechende Aufarbeitung liefert 20,5 g (das sind 92 % der Theorie) 1-Chlor-3,3-dimethyl-2-phenoxy-1,4-pentadien.

Beispiel A1 (c)

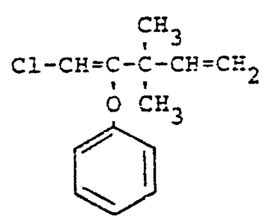

26,4 g (0,2 Mol) Kalium-phenolat und 16,5 g (0,1 Mol) 1,1-Dichlor-3,3-dimethyl-1,4-pentadien werden in 100 ml Hexamethylphosphorsäuretriamid während 6 Stunden auf 180 °C erhitzt. Die Beispiel A1 (a) analoge Aufarbeitung liefert 15,8 g (das sind 71 % der Theorie) 1-Chlor-3,3-dimethyl-2-phenoxy-1,4-pentadien.

Beispiel B1 (b)

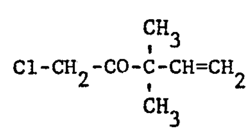

22,2 g (0,1 Mol) 1-Chlor-3,3-dimethyl-2-phenoxy-1,4-pentadien werden in 200 ml Ethanol und 50 ml konz. Salzsäure während 5 Stunden unter Rückfluß erhitzt. Die Aufarbeitung gemäß Beispiel B1 (a) führt zu 12 g 1-Chlor-3,3-dimethyl-pent-4-en-2-on, das sind 82 % der Theorie.

Beispiel B1 (c)

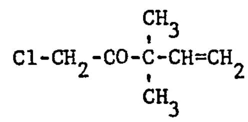

4,44 g (20 mMol) 1-Chlor-3,3-dimethyl-2-phenoxy-1,4-pentadien werden in 20 ml Dioxan und 10 ml 50 % Schwefelsäure während 6 Stunden auf 100 °C erwärmt. Die Aufarbeitung gemäß Beispiel B1 (a) liefert 2,1 g 1-Chlor-3,3-dimethyl-pent-4-en-2-on, das sind 72 % der Theorie.

Beispiel B1 (d)

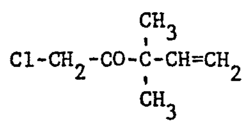

22,2 g (0,1 Mol) 1-Chlor-3,3-dimethyl-2-phenoxy-1,4-pentadien werden in 100 ml Essigsäure während 4 Stunden unter Rückfluß erhitzt. Die Beispiel B1 (a) entsprechende Aufarbeitung liefert 11,9 g 1-Chlor-3,3-dimethyl-pent-4-en-2-on, das sind 81 % der Theorie.

Beispiel B1 (e)

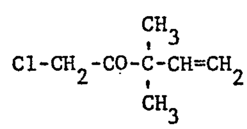

22,2 g (0,1 Mol) 1-Chlor-3,3-dimethyl-2-phenoxy-1,4-pentadien werden mit 100 ml 15 %iger Salzsäure unter Rückfluß erhitzt. Nach der Aufarbeitung gemäß Beispiel B1 (a) erhält man 11,4 g 1-Chlor-3,3-dimethyl-pent-4-en-2-on, das sind 78 % der Theorie.

Beispiel A2 (a)

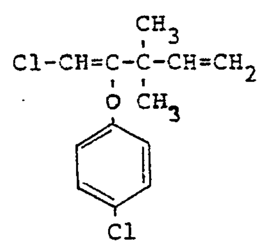

51,4 g (0,4 Mol) p-Chlorphenol werden in 300 ml Dimethylformamid mit 80 ml 30 %iger Natriummethylat-Lösung (0,4 Mol) umgesetzt, anschließend wird bei 30 mbar Lösungsmittel abgezogen. Dann werden 33 g (0,2 Mol) 1,1-Dichlor-3,3-dimethyl-1,4-pentadien zugesetzt und die Mischung während 9 Stunden unter Rückfluß erhitzt ; Aufarbeitung gemäß Beispiel A1 (a). Die Destillation bei Kp 0,2 mm Hg/122-130 °C liefert 44,8 g 1-Chlor-3,3-dimethyl-2-(p-chlorphenoxy)-1,4-pentadien, das sind 87 % der Theorie.

Beispiel B1 (f)

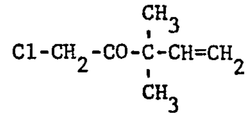

5,16 g (20 mMol) 1-Chlor-3,3-dimethyl-2-(p-chlorphenoxy)-1,4-pentadien, hergestellt nach obigem Beispiel A2 (a), werden im Gemisch aus 20 ml Ameisensäure und 2 ml konz. Salzsäure während 2 Stunden auf 50 °C erwärmt. Die Beispiel B1 (a) entsprechende Aufarbeitung liefert 2,47 g 1-Chlor-3,3-dimethyl-pent-4-en-2-on, das sind 84 % der Theorie.

Beispiel A3 (a)

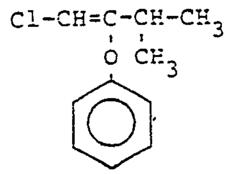

Zu der Suspension von 232 g (2 Mol) Natrium-phenolat in 1 l Dimethylformamid werden bei 150 °C langsam 139 g (1 Mol) 1,1-Dichlor-3-methyl-1-buten getropft und während 8 Stunden unter Rückfluß erhitzt. Nach Aufarbeitung gemäß Beispiel A1 (a) erhält man bei Kp 0,3 mm Hg/80-86 °C 165 g 1-Chlor-3-methyl-2-phenoxy-1-buten, das sind 84 % der Theorie.

NMR ($CDCl_3$) : δ 1,05 (d, J = 6,5 Hz, 6H), 2,45 (h, J = 6,5 Hz, 1H), 5,75 (s, 1H), 6,8-7,4 (m, 5H).

Beispiel B2 (a)

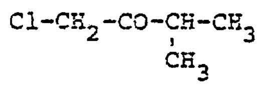

146,7 g (0,75 Mol) 1-Chlor-3-methyl-2-phenoxy-1-buten, hergestellt nach obigem Beispiel A3 (a), werden mit 375 ml Ameisensäure und 27,5 ml konz. Salzsäure während 1 Stunde auf 50 °C erwärmt. Die Aufarbeitung gemäß Beispiel B1 (a) führt zu 70,9 g 1-Chlor-3-methyl-2-butanon vom Kp 20 mm Hg/50-51 °C, das sind 78 % der Theorie.

NMR ($CDCl_3$) : δ 1,15 (d, H = 6,5 Hz, 6H), 2,9 (h, J = 6,5 Hz, 1H), 4,25 (s, 2H).

Beispiel A4 (a)

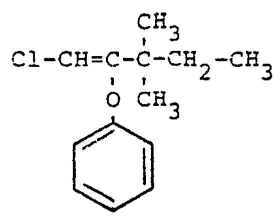

23,2 g (0,2 Mol) Natrium-phenolat werden mit 18,6 g (0,11 Mol) 1,1-Dichlor-3,3-dimethyl-1-penten in 100 ml Dimethylformamid während 20 Studen unter Rückfluß erhitzt. Nach üblicher Aufarbeitung destilliert man 20,4 g 1-Chlor-3,3-dimethyl-2-phenoxy-1-penten beim Kp 0,2 mm Hg/104 °C, das sind 84 % der Theorie.

NMR ($CDCl_3$) : δ 0,85 (t, J = 7 Hz, 3H), 1,05 (s, 6H), 1,5 (q, J = 7 Hz, 2H), 5,9 (s, 1H), 6,7-7,3 (m, 5H).

Beispiel B3 (a)

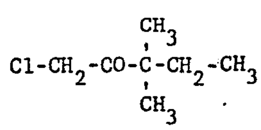

341 g (1,52 Mol) 1-Chlor-3,3-dimethyl-2-phenoxy-1-penten, hergestellt gemäß Beispiel A4 (a) oben, werden in 500 ml Ameisensäure und 100 ml konz. Salzsäure während 2 Stunden auf 80 °C erwärmt. Die übliche Aufarbeitung gemäß Beispiel B1 (a) führt zu 183 g 1-Chlor-3,3-dimethyl-2-pentanon vom Kp 20 mm Hg/75-83 °C, das sind 81 % der Theorie.

NMR ($CDCl_3$) : δ 0,8 (t, J = 7 Hz, 3H), 1,2 (s, 6H), 1,6 (q, J = 7 Hz, 2H), 4,35 (s, 2H).

Beispiel A5 (a)

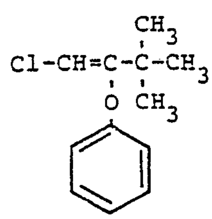

16,5 g (0,108 Mol) 1,1-Dichlor-3,3-dimethyl-1-buten werden mit 23,2 g (0,2 Mol) Natrium-phenolat in 100 ml Dimethylformamid während 12 Stunden unter Rückfluß erhitzt. Die übliche Aufarbeitung gemäß Beispiel A1 (a) liefert bei der Destillation beim Kp 0,5 mm Hg/80 °C 22,3 g 1-Chlor-3,3-dimethyl-2-phenoxy-1-buten.

NMR ($CDCl_3$) : δ 1,15 (s, 9H), 5,9 (s, 1H), 6,9-7,5 (m, 5H).

Beispiel B4 (a)

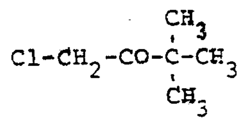

21 g (0,1 Mol) 1-Chlor-3,3-dimethyl-2-phenoxy-1-buten, hergestellt nach obigem Beispiel A5 (a), werden mit 20 ml Ameisensäure und 2 ml konz. Salzsäure während 1 Stunde auf 80 °C erwärmt. Die übliche Aufarbeitung gemäß Beispiel B1 (a) führt zu 12,1 g 1-Chlor-3,3-dimethyl-2-butanon vom Kp 24 mm Hg/70 °C, das sind 90 % der Theorie.

NMR ($CDCl_3$) : δ 1,2 (s, 9H), 4,45 (s, 2H).

Beispiel A6 (a)

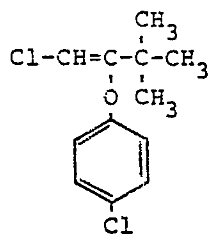

26 g (0,2 Mol) p-Chlorphenol werden mit 40 ml 30 % Natrium-methylat-Lösung (0,2 Mol) in 150 ml Dimethylformamid umgesetzt und bei 20 mbar vom Lösungsmittel befreit. Nach Zugabe von 15,3 g (0,1 Mol) 1,1-Dichlor-3,3-dimethyl-1-buten erhitzt man während 18 Stunden unter Rückfluß. Die Destillation nach Aufarbeitung gemäß Beispiel A1 (a) führt zu 21 g 1-Chlor-3,3-dimethyl-2-(p-chlorphenoxy)-1-buten vom Kp 0,15 mm Hg/120-128 °C.

NMR ($CDCl_3$) : δ 1,15 (s, 9H), 5,9 (s, 1H), 6,8-7,35 (m, 4 H).

Beispiel A7 (a)

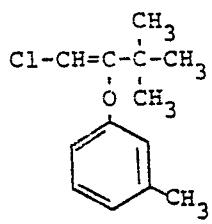

Analog Beispiel A6 (a) werden 0,2 Mol 3-Methylphenol und 0,1 Mol 1,1-Dichlor-3,3-dimethyl-1-buten zu 19,6 g 1-Chlor-3,3-dimethyl-2-(3′-methylphenoxy)-1-buten vom Kp 0,7 mm Hg/95-100 °C umgesetzt. Die Ausbeute beträgt 87 % der Theorie.

NMR ($CDCl_3$) : δ 1,15 (s, 9H), 2,3 (s, 3H), 5,85 (s, 1H), 6,6-7,3 (m, 4H).

Beispiel A8 (a)

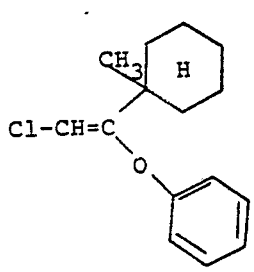

19,3 g (0,1 Mol) 1-(2',2'-Dichlorvinyl)-1-methyl-cyclohexan und 23,2 g (0,2 Mol) Natrium-phenolat werden in 100 ml Dimethylformamid während 10 Stunden unter Rückfluß erhitzt. Die übliche Aufarbeitung (s. A1 (a)) führt zu 18,6 g 1-Chlor-2-(1'-methylcyclohexyl)-2-phenoxyethylen vom Kp 0,03 mm Hg/100 °C. Die Ausbeute beträgt 74 % der Theorie.

NMR ($CDCl_3$) : δ 1,15 (s, 3H), 1,1-2,2 (m, 10H), 5,85 (s, 1H), 6,8-7,4 (m, 5H).

Beispiel B5 (a)

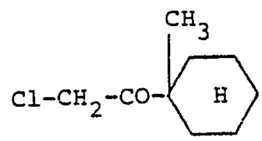

62 g (0,25 Mol) 1-Chlor-2-(1'-methylcyclohexyl)-2-phenoxy-ethylen, hergestellt nach A8 (a) oben, werden in 300 ml Ameisensäure und 60 ml konz. Salzsäure während 2 Stunden auf 80 °C erhitzt. Nach üblicher Aufarbeitung (s. B1 (a)) erhält man 39,6 g 1-Chloracetyl-1-methylcyclohexan vom Kp 20 mm Hg/115 °C. Ausbeute 92 % der Theorie.

NMR ($CDCl_3$) : δ 1,2 (s, 3H), 1,2-2,2 (m, 10H), 4,2 (s, 2H).

Desweiteren wurden in entsprechender Weise, wie in den obigen Beispielen angegeben, noch die folgenden Verbindungen hergestellt :

| Beispiel Nr. | | Kp (mm Hg/°C) |
|---|---|---|
| A9 (a) | 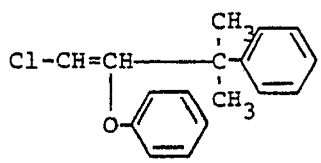 | 0,15/153-156 |
| B6 (a) | 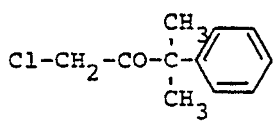 | 0,3/130-139 |
| A10 (a) | 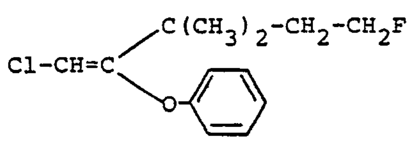 | 0,1/120-128 |
| B7 (a) | $Cl-CH_2-CO-C(CH_3)_2-CH_2-CH_2-F$ | 20/50-51 |
| A11 (a) | 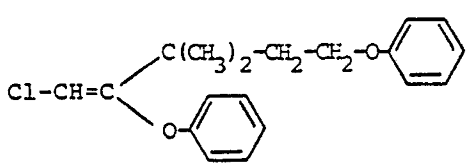 | 0,1/150-168 |
| B8 (a) | 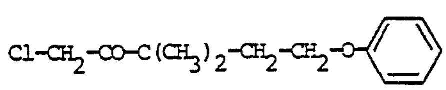 | 0,2/150-155 |
| A12 (a) | 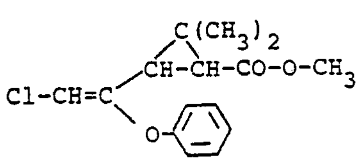 | 0,15/130-150 |
| B9 (a) | 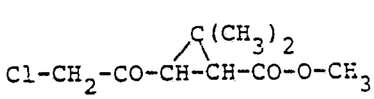 | 0,1/90 |

| Beispiel Nr. | | Kp (mm Hg/°C) |
|---|---|---|
| A13(a) | 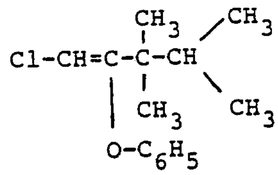 | 0,04/94-96° |
| B10(a) | 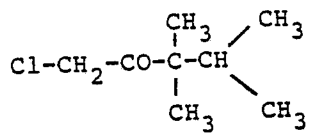 | 20/97-105° |
| A14 | 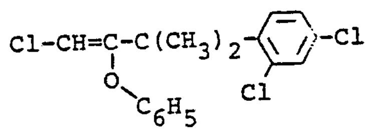 | 0,07/160-180° |
| B11 | 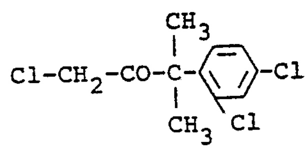 | 0,08/145-155° |
| A15 | 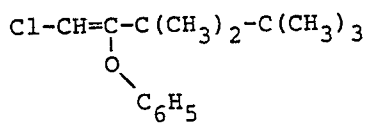 | 0,5/123-128° |
| B12 | 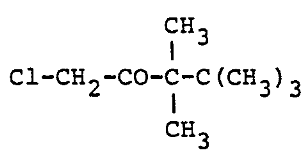 | 14/105-110° |
| A16 | 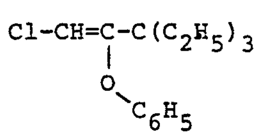 | 0,06/105-115° |
| B13 | $Cl-CH_2-CO-C(C_2H_5)_3$ | 18/102-107° |
| B14 | 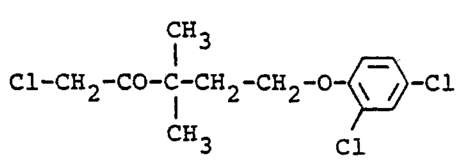 | Fp. 55-58° |

Die nachfolgend aufgeführten Verbindungen sind neu : Die Verbindungen gemäß Herstellungsbeispielen A1, A2, A3, A4, A8, A10, A11, A12, A13, A14, A15, A16 und B1, B3, B5, B7, B8, B9, B10, B11, B12, B13, B14.

Von besonderem Interesse ist die neue Verbindung B1 als Zwischenprodukt zur Herstellung von fungizid wirksamen Produkten ; die Endprodukte können in der weiter oben beschriebenen Weise hergestellt werden.

**Ansprüche**

1. Verfahren zur Herstellung von Monochlormethylketonen der allgemeinen Formel

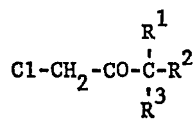

(I)

in welcher

$R^1$ und $R^2$ für Alkyl, Alkenyl, Alkinyl mit jeweils bis zu 4 Kohlenstoffatomen und für Phenyl stehen, wobei die genannten Gruppen gegebenenfalls durch Halogen, Phenyl, Alkoxy mit bis zu 3 Kohlenstoffatomen und gegebenenfalls chlorsubstituiertes Phenoxy substituiert sein können, ferner können auch

$R^1$ und $R^2$ gemeinsam für eine Alkylenkette mit 2 bis 7 Kohlenstoffatomen stehen, welche gegebenenfalls durch Methyl-Gruppen und/oder Alkoxycarbonyl-Gruppen mit 1 bis 3 Kohlenstoffatomen im Alkylteil substituiert sein kann, und

$R^3$ für Wasserstoff, Alkyl mit bis zu 10 Kohlenstoffatomen oder für Aryl mit 6 bis 10 Kohlenstoffatomen steht, wobei die Alkyl- und Aryl-Reste durch Halogen, Nitro, Alkoxy, Alkyl, Phenyl oder Cyano substituiert sein können,

dadurch gekennzeichnet, daß man 1,1-Dichloralkene der Formel

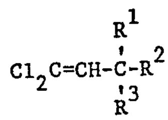

(II)

in welcher $R^1$, $R^2$ und $R^3$ die obengenannte Bedeutung besitzen, mit Phenolaten der Formel

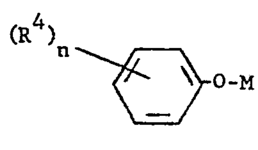

(III)

in welcher

$R^4$ für Chlor, Fluor, Alkyl und Alkoxy mit jeweils 1 bis 3 Kohlenstoffatomen, für Phenyl und/oder Nitro steht,

n für die Zahlen 0, 1, 2 oder 3 steht und

M für ein Äquivalent eines Alkalimetall- oder Erdalkalimetall-ions steht,

umsetzt und hernach das dabei erhaltene Reaktionsprodukt einer sauren Hydrolyse unterwirft.

2. Verfahren nach Anspruch 1, dadurch gekennzeichnet, daß man die Umsetzung im Temperaturbereich zwischen + 50 und 250 °C vornimmt.

3. Verfahren nach Ansprüchen 1 und 2, dadurch gekennzeichnet, daß man die Umsetzung im Temperaturbereich zwischen 100 und 200 °C vornimmt.

4. Verfahren nach Anspruch 1, dadurch gekennzeichnet, daß man den aus 1,1-Dichloralken der Formel (II) und Phenolat der Formel (III) erhaltenen Phenylether der Formel

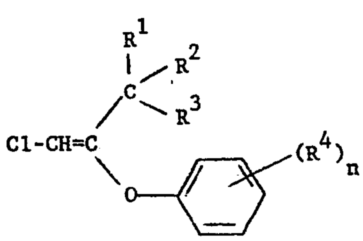

(IV)

in welcher

$R^1$, $R^2$, $R^3$, $R^4$ und n die in Anspruch 1 angegebene Bedeutung besitzen, isoliert, ehe man ihn der sauren Hydrolyse unterwirft.

5. 1-Chlor-3,3-dimethyl-pent-4-en-2-on der Formel

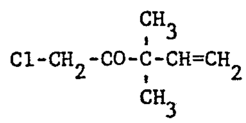

## Claims

1. Process for the preparation of monochloromethyl ketones of the general formula

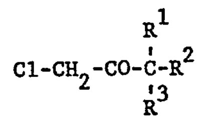

(I)

in which

$R^1$ and $R^2$ represent alkyl, alkenyl or alkinyl, with in each case up to 4 carbon atoms, or phenyl, it being possible for the stated groups to be optionally substituted by halogen, phenyl, alkoxy with up to 3 carbon atoms or optionally chlorine-substituted phenoxy, and, in addition,

$R^1$ and $R^2$ can also together represent an alkylene chain with 2 to 7 carbon atoms which can optionally be substituted by methyl groups and/or alkoxycarbonyl groups with 1 to 3 carbon atoms in the alkyl part, and

$R^3$ represents hydrogen, alkyl with up to 10 carbon atoms or aryl with 6 to 10 carbon atoms, it being possible for the alkyl and aryl radicals to be substituted by halogen, nitro, alkoxy, alkyl, phenyl or cyano, characterised in that 1,1-dichloroalkenes of the formula

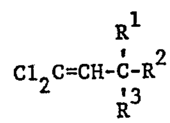

(II)

in which $R^1$, $R^2$ and $R^3$ have the above-mentioned meaning, are reacted with phenolates of the formula

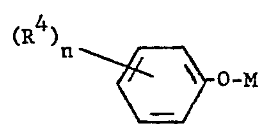

(III)

in which

$R^4$ represents chlorine, fluorine, alkyl and alkoxy with in each case 1 to 3 carbon atoms, phenyl and/or nitro,

n represents the numbers 0, 1, 2 or 3 and

M represents one equivalent of an alkali metal ion or alkaline earth metal ion,

and then the reaction product obtained thereby is subjected to an acid hydrolysis.

2. Process according to Claim 1, characterised in that the reaction is carried out in the temperature range of between + 50 and 250 °C.

3. Process according to Claims 1 and 2, characterised in that the reaction is carried out in the temperature range of between 100 and 200 °C.

4. Process according to Claim 1, characterised in that the phenyl ether, obtained from 1,1-dichloroalkene of the formula (II) and the phenolate of the formula (III), of the formula

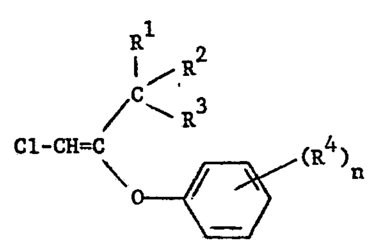

(IV)

in which $R^1$, $R^2$, $R^3$, $R^4$ and n have the meaning indicated in Claim 1, is isolated before being subjected to the acid hydrolysis.

5. 1-Chloro-3,3-dimethyl-pent-4-en-2-one of the formula

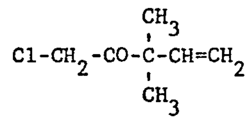

**Revendications**

1. Procédé de préparation de monochlorométhylcétones de formule générale

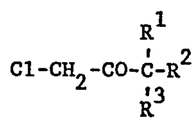 (I)

dans laquelle

$R^1$ et $R^2$ représentent des groupes alkyle, alcényle, alcynyle contenant chacun jusqu'à 4 atomes de carbone, ou phényle, les groupes en question pouvant, le cas échéant, être substitués par des halogènes, des groupes phényle, alcoxy contenant jusqu'à 3 atomes de carbone et phénoxy portant lui-même éventuellement des substituants chloro, ou bien encore

$R^1$ et $R^2$ forment ensemble une chaîne alkylène en $C_2$-$C_7$ éventuellement substituée par des groupes méthyle et/ou des groupes alcoxycarbonyle contenant 1 à 3 atomes de carbone dans la partie alkyle, et

$R^3$ représente l'hydrogène, un groupe alkyle contenant jusqu'à 10 atomes de carbone ou aryle contenant 6 à 10 atomes de carbone, les restes alkyle et aryle pouvant être substitués par des halogènes, des groupes nitro, alcoxy, alkyle, phényle ou cyano,

caractérisé en ce qu'on fait réagir des 1,1-dichloralcènes de formule

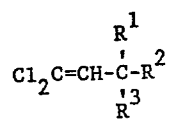 (II)

dans laquelle $R^1$, $R^2$ et $R^3$ ont les significations indiquées ci-dessus, avec des phénolates de formule

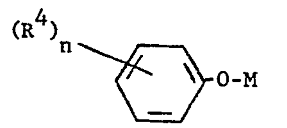 (III)

dans laquelle

$R^4$ représente le chlore, le fluor, un groupe alkyle ou alcoxy contenant chacun 1 à 3 atomes de carbone, un groupe phényle et/ou nitro,

n est égal à 0, 1, 2 ou 3, et

M représente un équivalent d'un ion de métal alcalin ou alcalinoterreux,

et on soumet ensuite le produit de réaction obtenu à une hydrolyse acide.

2. Procédé selon la revendication 1, caractérisé en ce que l'on effectue la réaction dans l'intervalle de température de + 50 à 250 °C.

3. Procédé selon les revendications 1 et 2, caractérisé en ce que l'on effectue la réaction dans l'intervalle de température de 100 à 200 °C.

4. Procédé selon la revendication 1, caractérisé en ce que l'on isole les éthers phényliques de formule

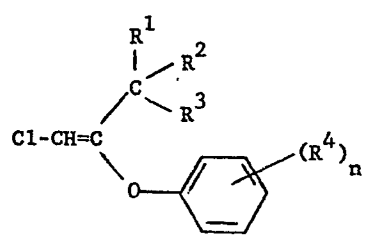 (IV)

dans laquelle $R^1$, $R^2$, $R^3$, $R^4$ et n ont les significations indiquées dans la revendication 1, obtenus à partir

du 1,1-dichloralcène de formule II et du phénolate de formule III, avant de les soumettre à l'hydrolyse acide.

5. La 1-chloro-3,3-diméthyl-penta-4-ène-2-one de formule

$$Cl-CH_2-CO-\underset{CH_3}{\overset{CH_3}{C}}-CH=CH_2$$